(19) **Europäisches Patentamt / European Patent Office / Office européen des brevets**

(11) **EP 2 554 988 B1**

(12) **EUROPEAN PATENT SPECIFICATION**

(45) Date of publication and mention of the grant of the patent:
**07.10.2015 Bulletin 2015/41**

(51) Int Cl.:
**G01N 33/564** (2006.01)   **G01N 33/543** (2006.01)
**G01N 33/571** (2006.01)

(21) Application number: **11765771.8**

(22) Date of filing: **31.03.2011**

(86) International application number:
**PCT/JP2011/058281**

(87) International publication number:
**WO 2011/125871 (13.10.2011 Gazette 2011/41)**

(54) **METHOD FOR STABILIZING GLYCEROPHOSPHOLIPIDS AND REAGENTS USING SAME**

VERFAHREN ZUR STABILISIERUNG VON GLYCEROPHOSPHOLIPIDEN UND REAGENZIEN DAMIT

MÉTHODE DE STABILISATION DE GLYCÉROPHOSPHOLIPIDES ET RÉACTIFS L'UTILISANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2010 JP 2010083681**

(43) Date of publication of application:
**06.02.2013 Bulletin 2013/06**

(73) Proprietor: **Sekisui Medical Co., Ltd.**
**Tokyo 103-0027 (JP)**

(72) Inventors:
• **ABE, Takayuki**
  **Ryugasaki-shi**
  **Ibaraki 301-0852 (JP)**
• **OTA, Tetsuya**
  **Ryugasaki-shi**
  **Ibaraki 301-0852 (JP)**

(74) Representative: **Hartz, Nikolai**
**Wächtershäuser & Hartz**
**Patentanwaltspartnerschaft mbB**
**Weinstrasse 8**
**80333 München (DE)**

(56) References cited:
**JP-A- 6 148 193        JP-A- 7 103 980**
**JP-A- 10 282 096      JP-A- 2003 294 753**
**US-A1- 2006 147 342**

• **SHUNSUKE YACHITA: 'Densensei Kikanshien HI Test ni Okeru Hitokuiteki Inhibitor Jyokyo ni Tsuite' JOURNAL OF THE JAPANESE SOCIETY ON POULTRY DISEASES vol. 20, no. 4, December 1984, pages 186 - 192**

## Description

Field of the Invention

The present invention relates to an immunoassay reagent for assaying an analyte such as antiphospholipid antibodies and to a method for stabilizing glycerophospholipid incorporated into the reagent.

Background of the Invention

In our bodies, antiphospholipid antibodies are produced by the following two diseases. One is syphilis, which is caused by infection with *Treponema Pallidum* as a pathogen thereof. The other is antiphospholipid antibody syndrome, which is a type of autoimmune disease.

In both cases, antiphospholipid antibodies are produced by a lipid antigen predominantly containing cardiolipin, which is a type of phospholipid. Thus, a reagent employed for the diagnosis of the above disease contains cardiolipin.

Currently, several methods employing the aforementioned phospholipid are used as syphilis test methods. Such methods include the VDRL (Veneral Disease Research Lab.) method, employing carbon or powdered kaolin as a carrier; the RPR

(rapid plasma reagin) card test (Non-Patent Document 1) ; and the latex agglutination method which employs a latex composed of polystyrene copolymer and the like as a carrier and is conducted by a biochemical auto-analyzer (Patent Document 1).

Meanwhile, antiphospholipid antibody syndrome is detected by ELISA (Patent Document 2).

In these tests, serum samples, plasma samples, cerebrospinal fluid samples, and similar samples are used.

Prior Art Documents

Patent Documents

     Patent Document 1: JP-A-H07-103980
     Patent Document 2: JP-A-H06-148193
     Patent Document 3: JP-A-H10-282096

Non-Patent Documents

Non-Patent Document 1: Public Health Reports Vol. 75 (1960), 985-988

Summary of the Invention

Problems to be solved by the invention

Generally, when a reagent for diagnosing the presence or absence of a disease by optical measurement of immune agglutination caused by antigen-antibody reaction is employed, in some cases, measurements may be lower than the correct values due to the influence of interfering substances present in samples. This phenomenon can be confirmed by a considerable difference in measurement between the case in which an analyte is added to a solution such as physiological saline or buffer free from the influence of interfering substances present in samples and the case in which the analyte is added to a sample such as serum or plasma containing interfering substances.

This interference causes the following two problematic cases.

(1) When the amount of antibody or antigen in a sample is small, immune agglutination might proceed insufficiently due to interfering substances. In this case, the disease which the patient suffers may fail to be detected.

(2) When the amount of antibody or antigen in a sample is larger than the quantification limit of the assay reagent, for the accurate measurement, the sample is diluted with a diluent such as physiological saline or serum so that the amount of antibody or antigen is reduced to fall within a measurable range, and the correct amount of antibody or antigen is calculated by multiplying the dilution factor by the value obtained from the diluted sample. Since physiological saline contains no interfering substance, there is a considerable difference in the measured amount of antibody or antigen between the case in which the sample is diluted with serum containing no antibody and the case in which the sample is diluted with physiological saline, which hinders accurate measurements.

The present inventors have conducted extensive studies to identify the interfering substance present in serum or plasma, and have found that the interfering substance is an endogenous lipoprotein. A further study by the inventors has also elucidated that the influence of the endogenous lipoprotein on the measurements can be avoided by adding a glycerophospholipid to the immune reaction system, whereby an antibody or an antigen contained in the sample can be determined with higher accuracy.

In a typical antiphospholipid antibody assay procedure, a water-soluble polymer is used as a sensitizer. The present inventors have also found that an assay reagent containing a glycerophospholipid has an impaired long-term storage stability when a typically employed polymer such as polyethylene glycol is incorporated thereinto.

Thus, an object of the present invention is to provide an accurate and stable immunoassay reagent using a glycerophospholipid and a method for stabilizing the reagent.

Means for solving the problems

Under such circumstances, the present inventors conducted further extensive studies in order to attain high stability of the immunoassay reagent using a glycerophospholipid and to obtain accurate measurements. As a result, the inventors have found that the long-term storage stability of the immunoassay reagent, which has never been attained through incorporation of polyethylene glycol, pullulan, a polymer containing 2-methacryloyloxyethylphospholylcholine, etc., can be remarkably improved by adding a polyvinylpyrrolidone to the assay reagent, and that the analyte can be assayed accurately. The present invention has been accomplished on the basis of these findings.

Accordingly, the present invention is directed to the following.

(1) A reagent for assaying an analyte in blood by immune reaction with a phospholipid antigen, wherein a glycerophospholipid and a polyvinylpyrrolidone are incorporated into the immune reaction system, as further defined in claim 1.
The glycerophospholipid is one or more species selected from the group consisting of phosphatidic acid, phosphatidylcholine, phosphatidylglycerol, phosphatidylethanolamine and phosphatidylserine.
The analyte is an antiphospholipid antibody, and the antibody is assayed by immune reaction with a phospholipid antigen. The phospholipid antigen is supported on an insoluble carrier.
(2) A reagent as described in (1) above, wherein the antiphospholipid antibody serving as an analyte is an anti-syphilis phospholipid antibody generated in blood through infection with syphilis.
(3) A reagent as described in (1) above, wherein the antiphospholipid antibody serving as an analyte is an antiphospholipid antibody generated in blood through antiphospholipid antibody syndrome, which is an autoimmune disease.
(4) A method for assaying an antibody present in blood as an analyte by immune reaction with a phospholipid antigen, wherein the method comprises incorporating a glycerophospholipid and polyvinylpyrrolidone into the immune reaction system.
The glycerophospholipid is one or more species selected from the group consisting of phosphatidic acid, phosphatidylcholine, phosphatidylglycerol, phosphatidylethanolamine, and phosphatidylserine.
The analyte is an antiphospholipid antibody, and the antibody is assayed by immune reaction with a phospholipid antigen.
The phospholipid antigen is supported on an insoluble carrier.
(5) An assay method as described in (4), wherein the antiphospholipid antibody serving as an analyte is an anti-syphilis phospholipid antibody generated in blood through infection with syphilis.

(6) An assay method as described in (4), wherein the antiphospholipid antibody serving as an analyte is an antiphospholipid antibody generated in blood through antiphospholipid antibody syndrome, which is an autoimmune disease.

Also described is a method for stabilizing a glycerophospholipid-containing liquid, wherein the method comprises incorporating polyvinylpyrrolidone into the glycerophospholipid-containing liquid and an agent for stabilizing a glycerophospholipid-containing liquid, the agent containing, as an active ingredient, polyvinylpyrrolidone.

Advantageous effect of the invention

According to the present invention, the influence of endogenous lipoprotein on immune reaction for assaying an analyte can be avoided, and there can be provided an assay reagent which has excellent storage stability and which exhibits its performance for a long period of time.

Brief Description of the Drawings

[Fig. 1] Fig. 1 is a graph showing the results of Test 1 of Comparative Referential Example 1, with the reaction system containing no glycerophospholipid.
[Fig. 2] Fig. 2 is a graph showing the results of Test 1 of Referential Example 2, with the reaction system containing 0.06 wt.% of hen's egg yolk-derived phosphatidylglycerol.
[Fig. 3] Fig. 3 is a graph showing the results of Test 2 of Referential Example 1, with the reaction system containing 0.12 wt.% of hen's egg yolk-derived phosphatidylglycerol.
[Fig. 4] Fig. 4 is a graph showing the results of Test 2 of Referential Example 2, with the reaction system containing 0.06 wt.% of hen's egg yolk-derived phosphatidylglycerol.
[Fig. 5] Fig. 5 is a graph showing the results of Test 2 of Referential Example 3, with the reaction system containing 0.03 wt.% of hen's egg yolk-derived phosphatidylglycerol.
[Fig. 6] Fig. 6 is a graph showing the results of Test 2 of Referential Example 4, with the reaction system containing 0.03 wt.% of a synthetic phosphatidylglycerol, 1,2-dimyristoyl-sn-glycero-3-phosphoglycerol sodium salt.
[Fig. 7] Fig. 7 is a graph showing the results of Test 2 of Referential Example 5, with the reaction system containing 0.06 wt.% of hen's egg yolk-derived phosphatidylethanolamine.
[Fig. 8] Fig. 8 is a graph showing the results of Test 2 of Referential Example 6, with the reaction system containing 0.03 wt.% of a synthetic phosphatidylcholine, 1,2-dimyristoyl-sn-glycero-3-phosphocholine.

[Fig. 9] Fig. 9 is a graph showing the results of Test 2 of Referential Example 7, with the reaction system containing 0.03 wt.% of a synthetic phosphatidylcholine, 1-palmitoyl-2-stearoyl-sn-glycero-3-phosphocholine.

[Fig. 10] Fig. 10 is a graph showing the results of Test 2 of Referential Example 8, with the reaction system containing 0.015 wt.% of hen's egg yolk-derived phosphatidylglycerol and 0.015 wt.% of hen's egg yolk-derived phosphatidylethanolamine.

[Fig. 11] Fig. 11 is a graph showing the results of Test 2 of Comparative Referential Example 1, with the reaction system containing no glycerophospholipid.

[Fig. 12] Fig. 12 is a table and a graph showing the results of Example 1, with the sample-diluent containing 0.43 mg/mL of hen's egg yolk-derived phosphatidylglycerol and 0.5 wt.% of polyvinylpyrrolidone.

[Fig. 13] Fig. 13 is a table and a graph showing the results of Example 2, with the sample-diluent containing 0.55 mg/mL of hen's egg yolk-derived phosphatidylglycerol and 0.5 wt.% of polyvinylpyrrolidone.

[Fig. 14] Fig. 14 is a table and a graph showing the results of Example 3, with the sample-diluent containing 0.64 mg/mL of hen's egg yolk-derived phosphatidylglycerol and 0.5 wt.% of polyvinylpyrrolidone.

[Fig. 15] Fig. 15 is a table and a graph showing the results of Comparative Example 1, with the sample-diluent containing 0.43 mg/mL of hen's egg yolk-derived phosphatidylglycerol and 0.8 wt.% of pullulan.

[Fig. 16] Fig. 16 is a table and a graph showing the results of Comparative Example 2, with the sample-diluent containing 0.43 mg/mL of hen's egg yolk-derived phosphatidylglycerol and 0.5 wt.% of Lipidure.

[Fig. 17] Fig. 17 is a table and a graph showing the results of Comparative Example 3, with the sample-diluent containing 0.43 mg/mL of hen's egg yolk-derived phosphatidylglycerol and 0.6 wt.% of polyethylene glycol.

Modes for Carrying Out the Invention

The present invention is directed to a reagent for assaying an analyte in blood by immune reaction with an antigen, wherein a glycerophospholipid and a polyvinylpyrrolidone are incorporated into the immune reaction system, and a method for determining an analyte with the reagent.

The reagent of the present invention contains a glycerophospholipid for avoiding the influence of an endogenous lipoprotein and polyvinylpyrrolidone for improving the storage stability of a liquid containing the glycerophospholipid.

The glycerophospholipid is selected from phosphatidic acid, phosphatidylcholine, phosphatidylglycerol, phosphatidylethanolamine, and phosphatidylserine. Of these, phosphatidic acid, phosphatidylcholine, phosphatidylglycerol, and phosphatidylserine are preferred.

The glycerophospholipid is preferably incorporated into the reaction system at a concentration of 0.005 to 0.20 wt.%, more preferably 0.015 to 0.12 wt.%. However, no particular limitation is imposed on the glycerophospholipid concentration, in view of the fact that the amount of glycerophospholipid should be appropriately adjusted depending on the amount of sample to be analyzed.

The glycerophospholipid shall be present in the reaction system such as in a sample-diluent or in a reagent containing an antibody or an antigen. The glycerophospholipid is preferably incorporated into a sample-diluent for the purpose of avoiding the influence of endogenous lipoprotein.

The phosphatidic acid may originate from animals or plants and can be generally produced through decomposition of phosphatidylcholine or phosphatidylglycerol with phospholipase A2.

No particular limitation is imposed on the number of carbon atoms and the unsaturation degree of each of the two acyl groups in the phosphatidic acid. Generally, the acyl group has 10 to 18 carbon atoms and 0 to 2 unsaturated bonds. In addition, the two acyl groups are not necessarily equal in number of carbon atoms and of unsaturated bonds. The two acyl groups may be a combination of those having different numbers of carbon atoms from each other, and may be a combination of two saturated acyl groups, a combination of a saturated acyl group and an unsaturated acyl group, or a combination of two unsaturated acyl groups. Generally, phosphatidic acids originating from animals and plants are in the form of a mixture of phosphatidic acid species bearing acyl groups having different number of carbon atoms and different unsaturation degrees.

The aforementioned phosphatidic acid may be a chemically synthesized product. Examples of commercial products thereof include sodium 1,2-dimyristoyl-sn-glycero-3-phosphatidate, sodium 1,2-dipalmitoyl-sn-glycero-3-phosphatidate, and sodium 1,2-distearoyl-sn-glycero-3-phosphatidate.

The phosphatidylcholine may originate from animals or plants and is generally selected from phosphatidylcholines purified from soybean or hen's egg yolk.

No particular limitation is imposed on the number of carbon atoms and the unsaturation degree of each of the two acyl groups in the phosphatidylcholine. Generally, the acyl group has 10 to 22 carbon atoms and 0 to 2 unsaturated bonds. In addition, the two acyl groups are not necessarily equal in number of carbon atoms and of unsaturated bonds. The two acyl groups may be a combination of those having different numbers of carbon atoms from each other, and may be a combination of two saturated acyl groups, a combination of a saturated acyl group and an unsaturated acyl group, or a combination of two unsaturated acyl groups. Generally, phosphatidylcholines originating from animals and plants are in the form of a mixture of phosphatidylcholine species bearing acyl groups having different number of carbon atoms and different unsaturation degrees.

The aforementioned phosphatidylcholine may be a chemically synthesized product. Examples of commercial products thereof include 1,2-didecanoyl-sn-glycero-3-phosphocholine, 1,2-dimyristoyl-sn-glycero-3-phosphocholine, 1,2-dipalmitoyl-sn-glycero-3-phosphocholine, 1,2-distearoyl-sn-glycero-3-phosphocholine, 1,2-dioleoyl-sn-glycero-3-phosphocholine, 1,2-dilinoleoyl-sn-glycero-3-phosphocholine, 1,2-diercoyl-sn-glycero-3-phosphocholine, 1-myristoyl-2-palmitoyl-sn-glycero-3-phosphocholine, 1-myristoyl-2-stearoyl-sn-glycero-3-phosphocholine, 1-myristoyl-2-oleoyl-sn-glycero-3-phosphocholine, 1-palmitoyl-2-oleoyl-sn-glycero-3-phosphocholine, 1-stearoyl-2-oleoyl-sn-glycero-3-phosphocholine, 1-palmitoyl-2-myristoyl-sn-glycero-3-phosphocholine, and 1-palmitoyl-2-stearoyl-sn-glycero-3-phosphocholine.

The phosphatidylglycerol may originate from animals or plants and is generally selected from phosphatidylglycerols purified from soybean or hen's egg yolk.

No particular limitation is imposed on the number of carbon atoms and the unsaturation degree of each of the two acyl groups in the phosphatidylglycerol. Generally, the acyl group has 10 to 22 carbon atoms and 0 to 2 unsaturated bonds. Also, the number of carbon atoms and the unsaturation degree of one acyl group are not necessarily equal to those of the other acyl group. The two acyl groups may be those having different numbers of carbon atoms, and a combination of two saturated acyl groups, a combination of a saturated acyl group and an unsaturated acyl group, or a combination of two unsaturated acyl groups may be employed. Generally, phosphatidylglycerols originating from animals and plants are in the form of a mixture of phosphatidylglycerol species bearing acyl groups having different number of carbon atoms and different unsaturation degrees.

The aforementioned phosphatidylglycerol may be a chemically synthesized product. Examples of commercial products thereof include 1,2-dimyristoyl-sn-glycero-3-phosphoglycerol sodium salt, 1,2-dimyristoyl-sn-glycero-3-phosphoglycerol ammonium salt, 1,2-dipalmitoyl-sn-glycero-3-phosphoglycerol sodium salt, 1,2-dipalmitoyl-sn-glycero-3-phosphoglycerol ammonium salt, 1,2-distearoyl-sn-glycero-3-phosphoglycerol sodium salt, 1,2-distearoyl-sn-glycero-3-phosphoglycerol ammonium salt, 1,2-dioleoyl-sn-glycero-3-phosphoglycerol sodium salt, 1,2-diercoyl-sn-glycero-3-phosphoglycerol sodium salt, and 1-palmitoyl-2-oleoyl-sn-glycero-3-phosphoglycerol sodium salt.

The phosphatidylethanolamine may originate from animals or plants and is generally selected from phosphatidylethanolamines purified from soybean or hen's egg yolk.

No particular limitation is imposed on the number of carbon atoms and the unsaturation degree of each of the two acyl groups in the phosphatidylethanolamine. Generally, the acyl group has 10 to 22 carbon atoms and 0 to 2 unsaturated bonds. Also, the number of carbon atoms and the unsaturation degree of one acyl group are not necessarily equal to those of the other acyl group. The two acyl groups may be those having different numbers of carbon atoms, and a combination of two saturated acyl groups, a combination of a saturated acyl group and an unsaturated acyl group, or a combination of two unsaturated acyl groups may be employed. Generally, phosphatidylethanolamines originating from animals and plants are in the form of a mixture of phosphatidylethanolamine species bearing acyl groups having different number of carbon atoms and different unsaturation degrees.

The aforementioned phosphatidylethanolamine may be a chemically synthesized product. Examples of commercial products thereof include 1,2-dimyristoyl-sn-glycero-3-phosphoethanolamine, 1,2-dipalmitoyl-sn-glycero-3-phosphoethanolamine, 1,2-distearoyl-sn-glycero-3-phosphoethanolamine, 1,2-dilinoleoyl-sn-glycero-3-phosphoethanolamine, and 1,2-diercoyl-sn-glycero-3-phosphoethanolamine.

The phosphatidylserine may originate from animals or plants and is generally selected from phosphatidylserines purified from soybean or hen's egg yolk.

No particular limitation is imposed on the number of carbon atoms and the unsaturation degree of each of the two acyl groups in the phosphatidylserine. Generally, the acyl group has 10 to 22 carbon atoms and 0 to 2 unsaturated bonds. Also, the number of carbon atoms and the unsaturation degree of one acyl group are not necessarily equal to those of the other acyl group. The two acyl groups may be those having different numbers of carbon atoms, and a combination of two saturated acyl groups, a combination of a saturated acyl group and an unsaturated acyl group, or a combination of two unsaturated acyl groups may be employed. Generally, phosphatidylserines originating from animals and plants are in the form of a mixture of phosphatidylserine species bearing acyl groups having different number of carbon atoms and different unsaturation degrees.

The aforementioned phosphatidylserine may be a chemically synthesized product. Examples of commercial products thereof include 1,2-dimyristoyl-sn-glycero-3-phospho-L-serine sodium salt, 1,2-dipalmitoyl-sn-glycero-3-phospho-L-serine sodium salt, 1,2-distearoyl-sn-glycero-3-phospho-L-serine sodium salt, and 1,2-dioleoyl-sn-glycero-3-phospho-L-serine sodium salt.

The aforementioned glycerophospholipid is dissolved or dispersed in the buffer for the reaction system. A scarcely soluble glycerophospholipid may be dissolved or dispersed through ultrasonication. Alternatively, a surfactant may be added to dissolve such a glycerophospholipid.

No particular limitation is imposed on the surfactant generally employed above, so long as it can solubilize the lipid. Examples of preferred surfactants include sucrose fatty acid esters such as sucrose monolaurate; alkylglycosides such as lysophosphatidylcholine, octylglucoside, and dodecylmaltoside; and dextran sulfate.

In the present invention, there may be used a polyvinylpyrrolidone which has been industrially synthesized and which has a molecular weight of about 10,000 to about 360,000. The viscosity index of the polyvinylpyrrolidone, represented by K value, is preferably 30 or higher, more preferably 60 or higher.

As used herein, the index K value of polyvinylpyrrolidone refers to a value generally serving as an index for molecular weight. Specifically, the K value is obtained by measuring the relative viscosity of 1 wt.% aqueous polyvinylpyrrolidone solution at 25°C by means of a capillary viscometer, inputting the relative viscosity value to the following Fikentscher's viscosity equation:

[F1]

$$\log_{10}\eta_{rel} = \left[ \frac{75K_0^2}{1+1.5K_0 c} + K_0 \right] c$$

(wherein $\eta_{rel}$ represents relative viscosity, c represents concentration of aqueous solution (g/100 mL) (i.e., the amount of polyvinylpyrrolidone (g) contained in the aqueous solution (100 mL)), and $k_0$ represents variable in relation to K value), and multiplying the thus-obtained $k_0$ value by 1,000. The larger the K value, the higher the molecular weight.

the aforementioned polyvinylpyrrolidone concentration can be appropriately determined depending on the type of analyte, in view of the fact as described in Patent Document 3 that polyvinylpyrrolidone also serves as a sensitizer.

From the viewpoints of storage stability and sensitizing effect, generally, the polyvinylpyrrolidone concentration is preferably 0.05 to 2 wt.%, with respect to the total amount of the reaction system (i.e., liquid), more preferably 0.1 to 1.0 wt.%.

Polyvinylpyrrolidone is preferably incorporated into the glycerophospholipid-containing liquid, for the purpose of enhancement in storage stability of the glycerophospholipid-containing liquid.

In the case where the amount of analyte determined by immune reaction is small, a sensitizer is added for promoting immune agglutination. There have been already known compounds serving as such a sensitizer; such as polyethylene glycol, pullulan, polyacrylic acid, dextran sulfate, polyvinylpyrrolidone, carboxymethylcellulose, and 2-methacryloyloxyethylphospholylcholine-methacrylic acid copolymer. However, quite surprisingly, the inventors have found that, among these sensitizers, polyvinylpyrrolidone exhibits a property of increasing the storage stability of glycerophospholipid-containing liquid, in addition to the sensitizing action.

No particular limitation is imposed on the analyte of the present invention, so long as it is an anti-phospholipid antibody present in blood. An example of a typically known analyte is an anti-*Treponema pallidum* antibody.

Examples of the antiphospholipid antibody include an anti-syphilis phospholipid antibody, which emerges in blood through infection with syphilis, and an antiphospholipid antibody, which emerges in blood through infection with antiphospholipid antibody syndrome (i.e., an autoimmune disease).

In the method and assay reagent of the present invention, the antibody is subjected to immune reaction with an antigen. Thus, the assay reagent of the present invention generally contains an antigen which reacts with the target analyte via immune reaction. A phospholipid is used as the antigen.

As the phospholipid serving as the phospholipid antigen, three phospholipids, i.e., cardiolipin, phosphatidylcholine, and cholesterol, are generally employed. However, it is not necessarily the case that all three phospholipids are contained in the reagent, and any of the three can be selected with respect to the disease to be detected. For example, the reagent for detecting antiphospholipid antibody syndrome (i.e., an autoimmune disease) contains at least cardiolipin, and the anti-syphilis phospholipid antibody assaying reagent contains at least cardiolipin and phosphatidylcholine. Phosphatidylcholine and cholesterol are often used with cardiolipin as a mixture for the enhancement of specificity and sensitivity. The proportions by weight among three phospholipids: cardiolipin :

phosphatidylcholine : cholesterol are preferably about 1 : (3 to 30) : (0 to 10), more preferably about 1 : (3 to 30) : (0.5 to 10). However, the proportions are not limited thereto and are appropriately adjusted depending on the purpose of the reagent.

The phospholipid may be obtained from animals and plants or chemically synthesized, and the production method is appropriately chosen depending on the purpose. Generally, cardiolipin which is extracted and purified from cow's heart, phosphatidylcholine which is extracted and purified from hen's egg yolk, cholesterol which is extracted from wool, and all of these three compounds which are sythesized can be used. Alternatively, these phospholipids employed in the invention may be synthesized products or commercial products.

Before use, the aforementioned antigens such as a phospholipid antigen are generally dispersed in an appropriate solution. No particular limitation is imposed on the solution, and phosphate buffer, Tris-HCl buffer, glycine buffer, etc. may be employed. In dispersing such an antigen in the solution, the antigen is dispersed by causing it to be supported on an insoluble carrier, or by forming liposome.

In the present invention, there may be used, as the insoluble carrier, microparticle carriers which have been conventionally and generally employed in immunological agglutination reaction and agglutination inhibition reaction. Among these microparticle carriers, preferred is a latex carrier formed of a synthetic polymer which can be mass-produced on an industrial scale. Examples of the synthetic polymer include polystyrene, styrene-sulfonic acid copolymer, styrene-methacrylic acid copolymer, acrylonitrile-butadienestyrene copolymer, vinyl chloride-acrylate ester copolymer, and vinyl acetate-acrylate ester copolymer. Of these, polystyrene and styrene-sulfonic acid copolymer are particularly preferred, in view of the fact that these polymers are excellent in absorption of phospholipid and can stably maintain biological activity during a long-term storage period. Other than the polymer carriers, there may be employed biological particles such as animal-derived erythrocytes and bacterial cells, and non-biological particles such as bentonite, collodion, cholesterol crystals, silica, kaolin, and carbon powder. The mean particle size of the insoluble carrier generally employed, which varies depending on the determination method and the measurement apparatus, is 0.1 to 1.0 $\mu$m as determined by means of a transmission electron microscope, preferably 0.1 to 0.5 $\mu$m.

No particular limitation is imposed on the method for causing the phospholipid antigen to be supported on the insoluble carrier. For example, the phospholipid antigen is caused to be supported on the insoluble carrier via physical and/or chemical bonding by using a conventionally known technique.

In a procedure of causing the phospholipid antigen or the antiphoshpolipid antibody to be physically supported on the insoluble carrier, a latex having a suitable particle size is mixed with a phospholipid dissolved in a suitable solvent (e.g., ethanol) (i.e., phospholipid latex mixture liquid) under stirring (sensitization step), and after passage over a specific period of time, the mixture is treated with a solution containing protein, sugar, peptide, etc. (blocking step), followed by dispersing it in an appropriate solvent.

Examples of the solvent in which the latex is dispersed include phosphate buffer, Tris-HCl buffer, and glycine buffer.

Examples of the sample analyzed through these test methods include blood samples, serum samples, plasma samples, and cerebrospinal fluid samples, which possibly contain the aforementioned analyte.

In the measurement procedure of the present invention, the degree of agglutination occurring from the immune reaction between the above-produced reagent and the antiphospholipid antibody present in the sample is optically measured, to thereby determine the amount of the antiphospholipid antibody present in the sample.

The agglutination degree is optically measured through a known technique. Examples of the technique include turbidimetry in which formation of agglutination is measured as an increase in turbidity; a method in which formation of agglutination is measured as a change in particle size distribution or mean particle size; and integrating-sphere optical turbidimetry in which a change in forward-scattered light attributed to formation of agglutination is measured by means of an integrating sphere, and the ratio of the change to the transmitted light intensity is analyzed. In any of the measurement techniques, at least two measurements are obtained at different points in time, and the degree of agglutination is obtained on the basis of the rate of increase in the measurements between the time points (rate assay). Alternatively, the measurement is performed at a certain point in time (typically, a conceivable end point of reaction), and the degree of agglutination is obtained on the basis of the measurement (end point assay). From the viewpoints of simplicity and speed of the measurement, the rate assay based on turbidimetry is preferably performed. In the optical measurement, there may be employed an optical instrument which can detect scattered light intensity, transmitted light intensity, absorbance, etc.; in particular, a generally employed automated analyzer. The measurement may be performed at a wavelength of 250 to 1000 nm, preferably 540 to 800 nm.

No particular limitation is imposed on the reaction temperature, so long as the aforementioned immune reaction occurs. The immune reaction is preferably performed at a constant temperature of 10 to 50°C, more preferably 10 to 40°C. The reaction time is appropriately adjusted.

No particular limitation is imposed on the reaction medium (liquid) of the reaction system where the immune reaction is performed, so long as the medium is an aqueous solution having a property which can satisfy physiological conditions under which the immune reaction can occur. Examples of the medium include phosphate buffer, citrate buffer, glycine buffer, Tris buffer, and Good's buffer. The reaction medium preferably has a pH of 5.5 to 8.5, more preferably 6.5 to 8.0. If needed, the reaction medium may further contain a stabilizer such as bovine serum albumin or sucrose; an antiseptic such as sodium azide; a salt-concentration-controlling agent such as sodium chloride; etc.

A glycerophospholipid-containing liquid can be stable for a long-term storage period by adding polyvinylpyrrolidone thereto. Such storage stability may be evaluated by an accelerated test. Generally, reagents containing a bio-substance such as a protein (e.g., antigen or antibody) or a lipid are stored at 2 to 10°C. The storage stability of such a reagent during a long period of time is estimated analogically through an accelerated test in which the sample is analyzed after stored typically at 20 to 40°C in a short period of time. The temperature of storage in the test is generally 37°C.

Examples

The present invention will next be described in detail by way of examples, which should not be construed as limiting the present invention thereto.

Referential Example 1

1) Preparation of lipid antigen liquid

A cardiolipin solution in ethanol (5 mg/mL, product of Sigma) (2 mL), a phosphatidylcholine (COATSOME NC-50, product of NOF Corporation) solution in ethanol (10 mg/mL) (10 mL), and a cholesterol (product of Nacalai Tesque) solution in ethanol (10 mg/mL) (3 mL) were mixed together, to thereby prepare a lipid antigen liquid.

2) Production of latex particles

Distilled water (1,100 g), styrene (200 g), sodium styrenesulfonate (0.2 g), and aqueous solution of potassium persulfate (1.5 g) dissolved in distilled water (50 g) were fed to a glass reactor (capacity: 2 L) equipped with a stirrer, a reflux condenser, a temperature sensor, a nitrogen conduit, and a jacket. The atmosphere of the reactor was changed to nitrogen, and then the mixture in the reactor was allowed to polymerize at 70°C under stirring for 48 hours.

After completion of polymerization, the reaction mixture was filtered through filter paper, to thereby recover latex particles. The mean particle size of the latex particles was determined by imaging the latex particles by means of a transmission electron microscope (JEM-1010, product of JEOL Ltd.) (magnification: 10,000). The image analysis was performed with respect to at least 100 particles. Thus, latex A having a mean particle size of 0.40 $\mu$m was produced.

3) Preparation of lipid antigen-sensitized latex reagent

The latex A (solid content: 10 wt.%) (100 $\mu$L), produced in 2) above, was gently stirred and maintained at 37°C. To the latex A, the aforementioned lipid antigen liquid (330 $\mu$L) was added in a batch manner, and the mixture was gently stirred at 37°C for 2 hours. Subsequently, 100mM phosphate buffered saline (100mM phosphate buffer (pH: 7.4) and NaCl 0.9 wt.%; hereinafter abbreviated as PBS) (2 mL) containing 1 wt.% bovine serum albumin (hereinafter abbreviated as BSA) (Fraction V, reagent grade, product of Millipore) was added in a batch manner, and the mixture was further stirred at 37°C for one hour. This product was centrifuged, to thereby remove the supernatant, and the precipitated latex was suspended again in 1% BSA-containing PBS. The purification procedure was repeated. Finally, the thus-purified latex was suspended in 100mM phosphate buffer (pH: 7.4) (hereinafter abbreviated as PB) (4 mL) containing 1 wt.% BSA, 7.5 wt.% choline chloride, 0.14 wt.% EDTA·2Na, and 0.1 wt.% sodium azide, to thereby produce a latex reagent.

4) Preparation of sample-diluent

BSA, pullulan (molecular weight: 200,000, product of Hayashibara), sodium azide, and an egg-yolk-derived phosphatidylglycerol (COATSOME NG-50LS, product of NOF Corporation) were added to 50mM phosphate buffer (pH: 7.4) such that the amounts of the ingredients were adjusted to 1 wt.%, 1.0 wt.%, 0.1 wt.%, and 0.17 wt.%, respectively, and the mixture was stirred. Under ice-cooling conditions, the mixture was subjected to ultrasonication for 30 minutes or longer by means of an ultrasonic crusher (power 20%, 0.25-inch microchip) until the mixture became a transparent solution, to thereby prepare a sample-diluent.

Referential Example 2

The procedure of "4) Preparation of sample-diluent" of Referential Example 1 was repeated, except that the egg-yolk-derived phosphatidylglycerol concentration was adjusted to 0.085 wt.%.

Referential Example 3

The procedure of "4) Preparation of sample-diluent" of Referential Example 1 was repeated, except that the egg-yolk-derived phosphatidylglycerol concentration was adjusted to 0.043 wt.%.

Referential Example 4

The procedure of "4) Preparation of sample-diluent" of Referential Example 1 was repeated, except that a synthetic phosphatidylglycerol--1,2-dimyristoyl-sn-glycero-3-phosphoglycerol sodium salt (COATSOME MG-4040, product of NOF Corporation)--was used at a concentration of 0.043 wt.%, instead of the egg-yolk-derived phosphatidylglycerol.

Referential Example 5

The procedure of "4) Preparation of sample-diluent" of Referential Example 1 was repeated, except that an egg-yolk-derived phosphatidylethanolamine (COATSOME NE-50, product of NOF Corporation) was used at a concentration of 0.085 wt.%, instead of the egg-yolk-derived phosphatidylglycerol.

Referential Example 6

The procedure of "4) Preparation of sample-diluent" of Referential Example 1 was repeated, except that a synthetic phosphatidylcholine--1,2-dimyristoyl-sn-glycero-3-phosphocholine (COATSOME MC-4040, product of NOF Corporation)--was used at a concentration of 0.043 wt.%, instead of the egg-yolk-derived phosphatidylglycerol, and that lysophosphatidylcholine (COATSOME MC-40H, product of NOF Corporation) serving as a surfactant was used at a concentration of 0.014 wt.%.

Referential Example 7

The procedure of "4) Preparation of sample-diluent" of Referential Example 1 was repeated, except that a synthetic phosphatidylcholine--1-palmitoyl-2-stearoyl-sn-glycero-3-phosphocholine (COATSOME MC-6080, product of NOF Corporation)--was used at a concentration of 0.043 wt.%, instead of the egg-yolk-derived phosphatidylglycerol, and that lysophosphatidylcholine (COATSOME MC-40H, product of NOF Corporation) serving as a surfactant was used at a concentration of 0.014 wt.%.

Referential Example 8

The procedure of "4) Preparation of sample-diluent" of Referential Example 1 was repeated, except that the egg-yolk-derived phosphatidylglycerol concentration was adjusted to 0.021 wt.%, and an egg-yolk-derived phosphatidylethanolamine was used at a concentration of 0.021 wt.%.

Comparative Referential Example 1

The procedure of "4) Preparation of sample-diluent" of Referential Example 1 was repeated, except that no glycerophospholipid was added.

The thus-produced reagents of Referential Examples 1 to 8 and Comparative Referential Examples 1 to 2 were tested, and the results are as follows.

(Test 1)

1) Fractionation of endogenous lipoprotein

Fractions containing high-density lipoprotein (hereinafter abbreviated as HDL) were recovered through potassium bromide density-gradient centrifugation.

In a specific procedure, 12 mL of 1.21-g/mL potassium bromide solution was dispensed into ultracentrifugation tubes (capacity: 50 mL). To each tube, syphilis-negative serum (10 mL) was slowly dispensed such that the serum was not intermingled with potassium bromide solution, to thereby form two strata. Then, in a similar manner, purified water (12 mL) was slowly dispensed such that water was not intermingled with the serum, to form another stratum thereon.

The tubes were subjected to centrifugation by means of an ultra-centrifuge at 4°C and 44,000 rpm for 20 hours.

An injection needle was connected to a tube made of Teflon (registered trademark) and employed so that a solution can be sampled through suction by means of a peristaltic pump connected thereto. Specifically, the injection needle was slowly inserted into the bottom of each of the ultracentrifugation tubes after ultracentrifugation, and serum having potassium bromide density gradient was recovered through suction by means of the peristaltic pump. An aliquot (0.7 mL) was sampled as a fraction from each tube, and 43 fractions were recovered in total.

Through measurement of the HDL cholesterol levels of the 43 fractions, HDL was found to be contained in fractions No. 5 to No. 15. Fractions No. 9 to No. 13, corresponding to the highest HDL cholesterol level region, were combined and dialyzed against physiological saline in order to remove potassium bromide. After dialysis, the HDL cholesterol level was determined by use of Cholestest (registered trademark) N HDL (product of Sekisui Medical Co., Ltd.), and the level was found to be 40 mg/dL.

Thus, an HDL-containing physiological saline solution was produced.

2) Assay samples

A syphilis antiphospholipid antibody-positive sample having an antibody titer of 120 R.U. was stepwise diluted with the HDL-containing physiological saline obtained in 1) of Test 1 above, physiological saline, or serum. The unit R.U. is a unit of syphilis-positive antibody titer. A titer of 1 R.U. corresponds to unity in the RPR card test. When the titer is 1 R.U. or higher, the sample is diagnosed as syphilis positive. When the international standard sample is assayed, a titer of 1 R.U. corresponds to 0.4 IU.

3) Measurement of agglutination amount

Agglutination amount was determined by means of a biochemical auto-analyzer (Hitachi 7180). A sample-diluent (180 μL) prepared in Referential Example 2 or Comparative Referential Example 1 and each sample (20 μL) produced in 2) of Test 1 were mixed together in a cell of the biochemical auto-analyzer. The mixture was incubated at 37°C for 5 minutes. Subsequently, the latex reagent (60 μL) produced in 3) of Referential Example 1 was added to and mixed with the incubate, and the mixture was incubated at 37°C for 5 minutes. The absorbance of the sample at a measurement wavelength of 700 nm was measured immediately after addition of the latex reagent and five minutes after the addition, and the difference between two measurements was calculated by means of the auto-analyzer (hereinafter represented by $\Delta Abs \times 10,000$). The difference in absorbance corresponds to the amount of agglutination increased by immune reaction.

4) Results

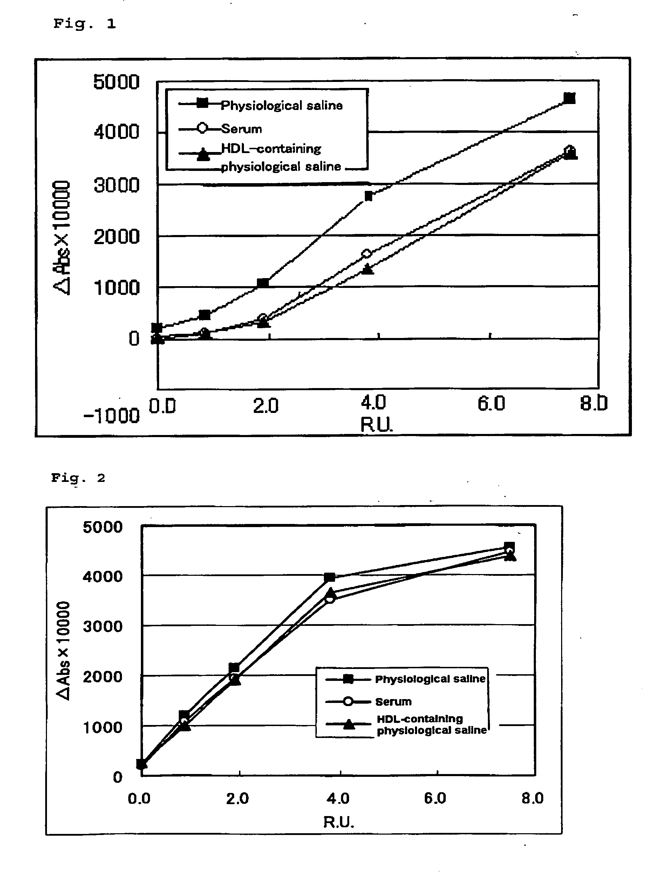

Figs. 1 and 2 show the results.

As shown in Fig. 1 and 2, in Comparative Referential Example 1, a drop in absorbance was observed when the HDL-containing physiological saline was used in a manner similar to the case in which serum was used. Thus, HDL present in serum was thought to be an interference component causing a drop in absorbance. In contrast Referential Example 2 in which a glycerophospholipid was added, a drop in absorbance as observed in Comparative Referential Example 1 was suppressed, when the sample was diluted with the HDL-containing physiological saline or serum, and the obtained absorbance was almost equivalent to that obtained by a similar sample diluted with HDL-free physiological saline. Therefore, the glycerophospholipid added in Referential Example 2 was found to suppress interference caused by HDL, to thereby suppress a drop in absorbance.

(Test 2)

1) Assay samples

A syphilis antiphospholipid antibody-positive sample having an antibody titer of 120 R.U. was stepwise diluted with physiological saline or serum.

2) Measurement of agglutination amount

Agglutination amount was determined by means of a biochemical auto-analyzer (Hitachi 7180). A sample-diluent (180 $\mu$L) prepared in any of Referential Examples 1 to 8 or Comparative Referential Example 1 and each sample (20 $\mu$L) produced in 1) of Test 1 were mixed together in a cell of the biochemical auto-analyzer. The mixture was incubated at 37°C for 5 minutes. Subsequently, the latex reagent (60 $\mu$L) produced in 1) of Referential Example 1 was added to and mixed with the incubate, and the mixture was incubated at 37°C for 5 minutes. The absorbance of the sample at a measurement wavelength of 700 nm was measured immediately after addition of the latex reagent and five minutes after the addition, and the difference between two measurements was calculated by means of the auto-analyzer (hereinafter represented by $\Delta$Abs$\times$10,000). The difference in absorbance corresponds to the amount of agglutination increased by immune reaction.

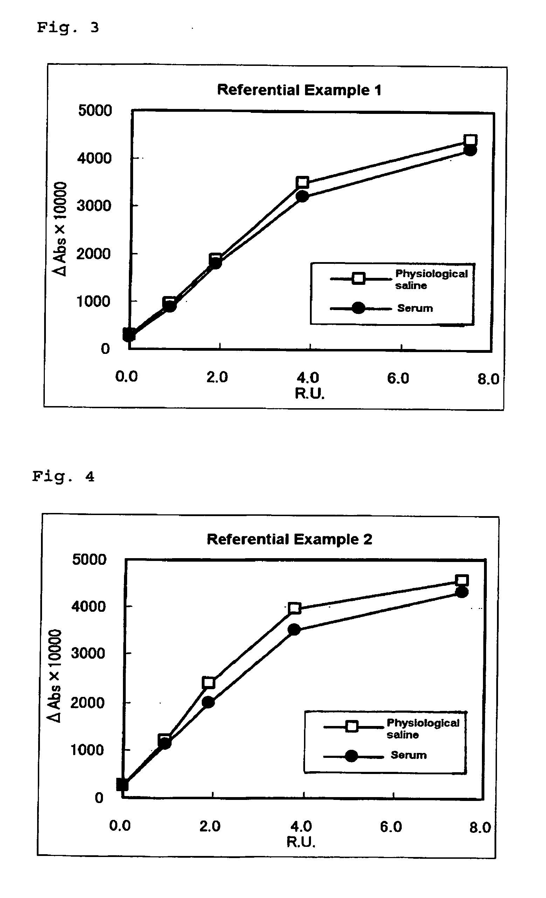

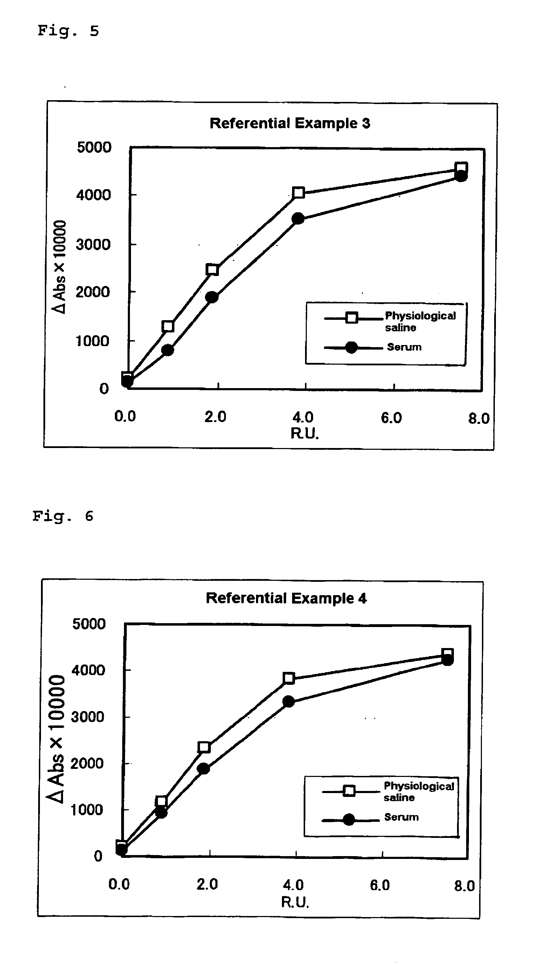

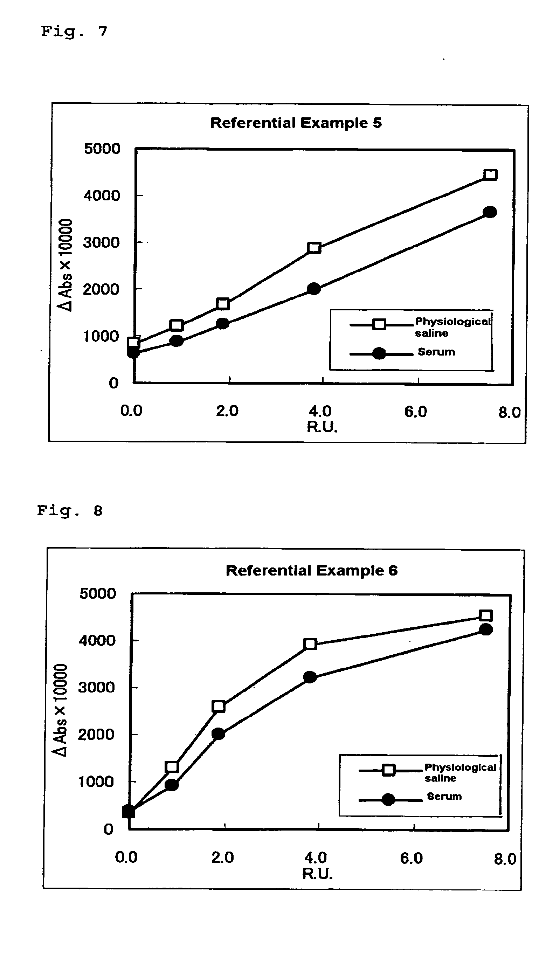

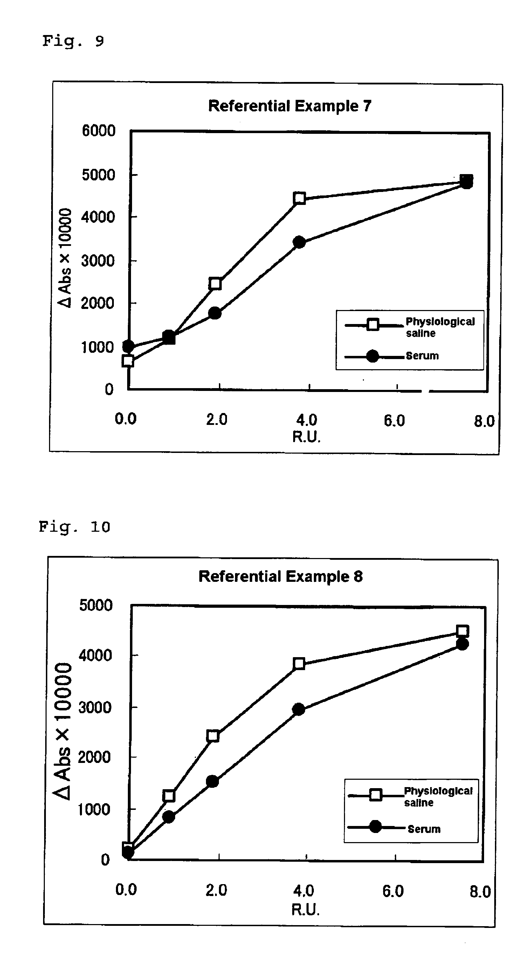

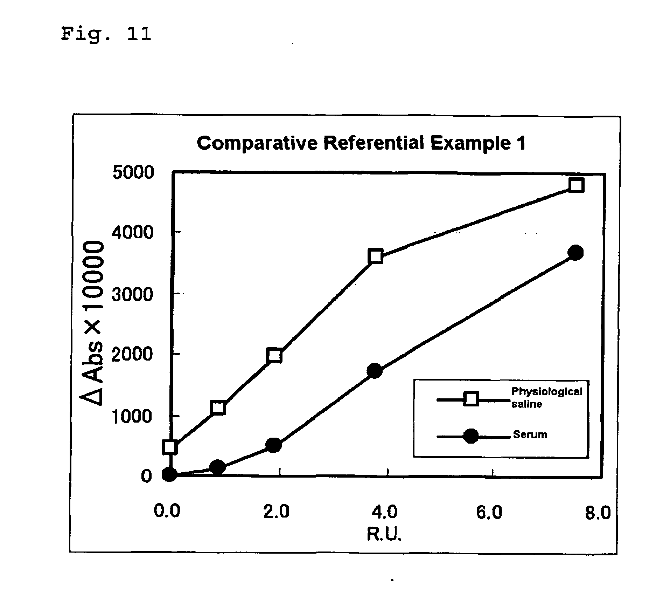

Figs. 3 to 11 show the results.

In Referential Examples 1 to 8, the difference in absorbance at any antibody concentration decreased when the sample was diluted with physiological saline or serum, as compared with Comparative Referential Example 1. Thus, the drop in absorbance, which would otherwise be caused by an interference component present in serum, was found to be mitigated through addition of any of the glycerophospholipids.

Example 1

1) Preparation of lipid antigen liquid

The same lipid antigen liquid as produced in 1) of Referential Example 1 was employed.

2) Production of latex particles

Distilled water (1,100 g), styrene (200 g), sodium styrenesulfonate (0.2 g), and aqueous solution of potassium persulfate (1.5 g) dissolved in distilled water (50 g) were fed to a glass reactor (capacity: 2 L) equipped with a stirrer, a reflux condenser, a temperature sensor, a nitrogen conduit, and a jacket. The atmosphere of the reactor was replaced with nitrogen, and then the mixture in the reactor was allowed to polymerize at 70°C under stirring for 48 hours.

After completion of polymerization, the reaction mixture was filtered through filter paper, to thereby recover latex particles. The mean particle size of the latex particles was determined by imaging the latex particles by means of a transmission electron microscope (JEM-1010, product of JEOL Ltd.) (magnification: 10,000$\times$). The image analysis was performed with respect to at least 100 particles. Thus, latex B having a mean particle size of 0.38 $\mu$m was produced.

3) Preparation of lipid antigen-sensitized latex reagent

The procedure of "Preparation of lipid antigen-sensitized latex reagent" described in 3) of Referential Example 1 was repeated, except that latex B was used instead of latex A.

4) Preparation of sample-diluent

BSA, Polyvinylpyrrolidone K90 (product of BASF), sodium azide, and an egg-yolk-derived phosphatidylglycerol (COATSOME NG-50LS, product of NOF Corporation) were added to 50mM phosphate buffer (pH: 7.4) such that the amounts of the ingredients were adjusted to 1 wt.%, 0.5 wt.%, 0.1 wt.%, and 0.43 mg/mL, respectively, and the mixture was stirred. Then, the mixture was subjected to ultrasonication for 30 minutes or longer by means of an ultrasonic crusher (power 20%, 0.25-inch microchip) until the mixture became a transparent solution, to thereby prepare a sample-diluent.

Example 2

The procedure of "4) Preparation of sample-diluent" of Example 1 was repeated, except that the egg-yolk-derived phosphatidylglycerol concentration was adjusted to 0.55 mg/mL.

Example 3

The procedure of "4) Preparation of sample-diluent" of Example 1 was repeated, except that the egg-yolk-derived phosphatidylglycerol concentration was adjusted to 0.64 mg/mL.

Comparative Example 1

The procedure of "4) Preparation of sample-diluent" of Example 1 was repeated, except that pullulan (molecular weight: 200,000, product of Hayashibara) was used at a concentration of 0.8 wt.% instead of polyvinylpyrrolidone.

Comparative Example 2

The procedure of "4) Preparation of sample-diluent" of Example 1 was repeated, except that Lipidure (2-methacryloyloxyethylphosphorylcholine-methacrylic acid copolymer: molecular weight 1,000,000, Lipidure-BL produced by NOF Corporation) was used at a concentration of 0.5 wt.% instead of polyvinylpyrrolidone.

Comparative Example 3

The procedure of "4) Preparation of sample-diluent" of Example 1 was repeated, except that polyethylene glycol (molecular weight: 70,000, product of Wako Pure Chemical Industries) was used at a concentration of 0.6 wt.% instead of polyvinylpyrrolidone.
The thus-produced reagents of Examples 1 to 3 and Comparative Examples 1 to 3 were tested, and the results are as follows.

1) 37°C Accelerated test

Each of the sample-diluents produced in Examples 1 to 3 and Comparative Examples 1 to 3 was placed in a bottle. The bottle was tightly closed and subjected to an accelerated test in an incubator at 37°C. At the end of storage day 3 and storage day 7, the bottle was taken from the incubator, and the agglutination amount was determined in through the procedure described in 3) below. The time-dependent change profile of the amounts of agglutination measured on day 0 (start of storage), day 3, and day 7 were analyzed. The latex reagent was stored at 4°C before use.

2) Assay samples RPR standard sera having five concentrations (0 R.U., 1 R.U., 2 R.U., 4 R.U., and 8 R.U.) (product of Sekisui Medical Co., Ltd.) were used.

3) Measurement of agglutination amount

The procedure of "3) Measurement of agglutination amount" of Test 1 was repeated, except that the sample-diluents produced in Referential Example 2 and Comparative Referential Example 1 and samples produced in 2) of Test 1 were changed to the sample-diluents (each 180 μL) produced in Examples 1 to 3 and Comparative Examples 1 to 3 and stored at 37°C in 1) above and the samples (each 20 μL) of 2) above.

3) Results

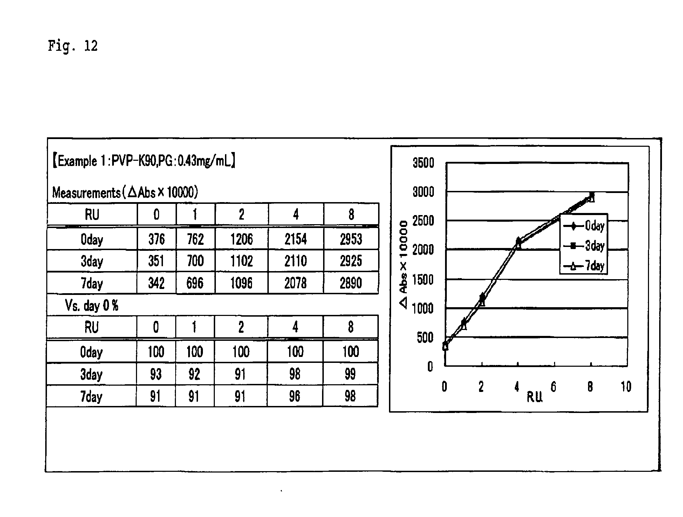

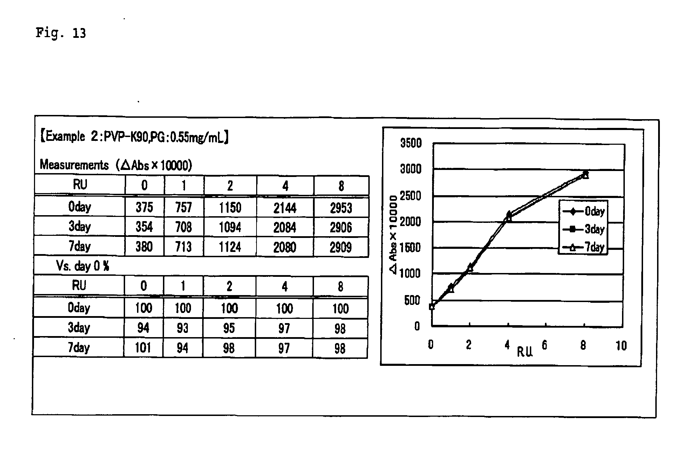

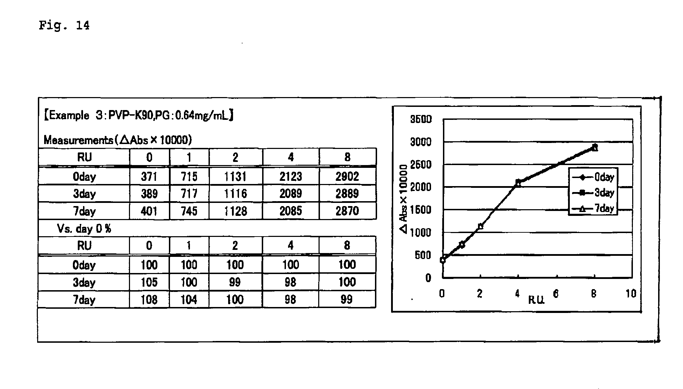

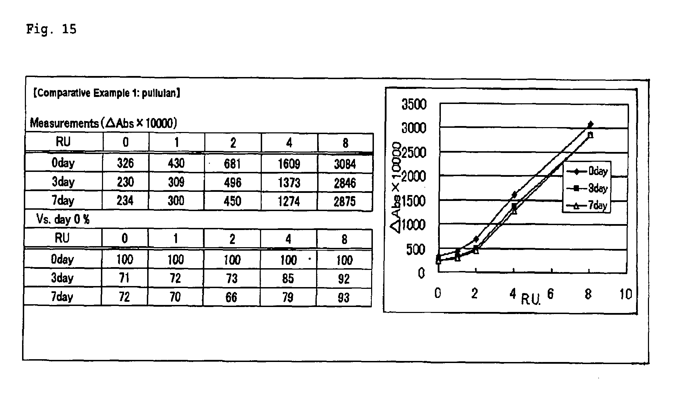

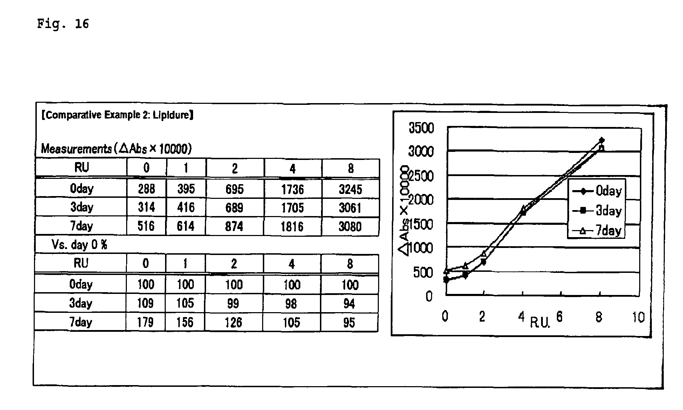

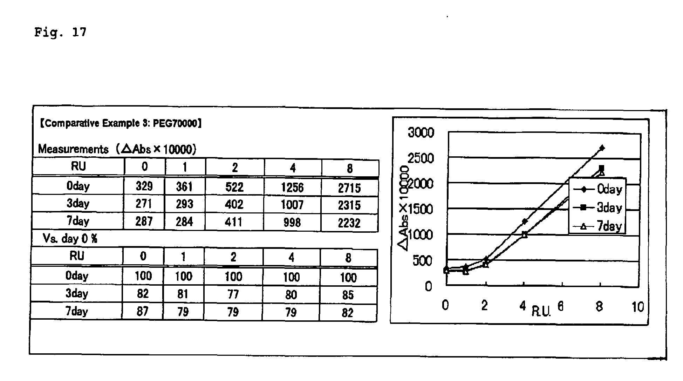

Figs. 12 to 17 show the results.
In Examples 1 to 3, the amounts of agglutination (measurements) measured at any antibody concentration on day 7 (storage at 37°C) were varied with respect to those on day 0 within a range of ±10%. In contrast in Comparative Example 1 to 3, the amounts of agglutination (measurements) measured at any antibody concentration on day 7 (storage at 37°C) were varied with respect to those on day 0 outside a range of ±10%. Therefore, polyvinylpyrrolidone was found to provide excellent storage stability as compared with other sensitizers.

Industrial Applicability

In a reagent for assaying an analyte present in blood by immune reaction, the reagent containing a glycerophospholipid for avoiding the interference of endogenous lipoprotein, incorporation of polyvinylpyrrolidone to the reagent attains excellent storage stability and a long-term performance.

**Claims**

1. A reagent for assaying an anti-phospholipid antibody as an analyte in blood by immune reaction with a phospholipid antigen which is supported on an insoluble carrier, wherein a glycerophospholipid and a polyvinylpyrrolidone are incorporated into the immune reaction system,
   wherein the glycerophospholipid is dissolved or dispersed in a buffer for the reaction system and is one or more species selected from the group consisting of phosphatidic acid, phosphatidylcholine, phosphatidylglycerol, phosphatidylethanolamine, and phosphatidylserine.

2. A reagent according to claim 1, wherein the anti-phospholipid antibody serving as an analyte is an anti-syphilis phospholipid antibody generated in blood through infection with syphilis.

3. A reagent according to claim 1, wherein the anti-phospholipid antibody serving as an analyte is an anti-phospholipid antibody generated in blood through anti-phospholipid antibody syndrome, which is an autoimmune disease.

4. A method for assaying an anti-phospholipid antibody present in blood as an analyte by immune reaction with a phospholipid antigen which is supported on an insoluble carrier, wherein the method comprises incorporating a glycerophospholipid and a polyvinylpyrrolidone into the immune reaction system, wherein the glycerophospholipid is dissolved or dispersed in a buffer for the reaction system and is one or more species selected from the group consisting of phosphatidic acid, phosphatidylcholine, phosphatidylglycerol, phosphatidylethanolamine, and phosphatidylserine.

5. An assay method according to claim 4, wherein the anti-phospholipid antibody serving as an analyte is an anti-syphilis phospholipid antibody generated in blood through infection with syphilis.

6. An assay method according to claim 4, wherein the anti-phospholipid antibody serving as an analyte is an anti-phospholipid antibody generated in blood through anti-phospholipid antibody syndrome, which is an autoimmune disease.

**Patentansprüche**

1. Ein Reagens zum Testen auf einen anti-Phospholipid-Antikörper als Analyt im Blut durch eine Immunreaktion mit einem Phospholipid-Antigen, das auf einem unlöslichen Träger geträgert ist, worin ein Phosphoglycerid und ein Polyvinylpyrrolidon im Immunreationssystem enthalten sind,
worin das Phosphoglycerid in einem Puffer für das Reaktionssystem gelöst oder dispergiert ist und ein oder mehr Element ausgewählt aus der Gruppe bestehend aus Phosphatidsäure, Phosphatidylcholin, Phosphatidylglycerin, Phosphatidylethanolamin und Phosphatidylserin ist.

2. Ein Reagens gemäß Anspruch 1, worin der anti-Phospholipid-Antikörper, der als Analyt dient, ein anti-Syphillisphospholipid-Antikörper ist, der im Blut durch Infektion mit Syphillis erzeugt wurde.

3. Ein Reagens gemäß Anspruch 1, worin der anti-Phospholipid-Antikörper, der als Analyt dient, ein anti-Phospholipid-Antikörper ist, der im Blut durch anti-Phospholipid-Antikörper-Syndrom, das eine Autoimmunerkrankung ist, erzeugt wurde.

4. Verfahren zum Testen auf einem anti-Phospholipid-Antikörper als Analyt in Blut mittels Immunreaktion mit einem Phospholipid-Antigen, das auf einem unlöslichen Träger geträgert ist, wobei das Verfahren umfasst ein Inkorporieren eines Phosphoglycerids und eines Polyvinylpyrrolidon in das Immunreationssystem,
worin das Phosphoglycerid in einem Puffer für das Reaktionssystem gelöst oder dispergiert ist und ein oder mehr Element ausgewählt aus der Gruppe bestehend aus Phosphatidsäure, Phosphatidylcholin, Phosphatidylglycerin, Phosphatidylethanolamin und Phosphatidylserin ist.

5. Ein Testverfahren gemäß Anspruch 4, worin der anti-Phospholipid-Antikörper, der als Analyt dient, ein anti-Syphillisphospholipid-Antikörper ist, der im Blut durch Infektion mit Syphillis erzeugt wurde.

6. Ein Testverfahren gemäß Anspruch 4, worin der anti-Phospholipid-Antikörper, der als Analyt dient, ein anti-Phospholipid-Antikörper ist, der in Blut durch anti-Phospholipid-Antikörper-Syndrom, das eine Autoimmunerkrankung ist, erzeugt wurde.

**Revendications**

1. Réactif pour doser un anticorps anti-phospholipide en tant qu'analyte dans le sang par réaction immunitaire avec un antigène phospholipidique qui est supporté sur un support insoluble, dans lequel un glycérophospholipide et une polyvinylpyrrolidone sont incorporés dans le système réactionnel immunitaire,
dans lequel le glycérophospholipide est dissous ou dispersé dans un tampon pour le système réactionnel et est une ou plusieurs espèce(s) choisie(s) dans l'ensemble constitué par l'acide phosphatidique, la phosphatidylcholine, le phosphatidylglycérol, la phosphatidyléthanolamine, et la phosphatidylsérine.

2. Réactif selon la revendication 1, dans lequel l'anticorps anti-phospholipide servant d'analyte est un anticorps anti-phospholipide de syphilis généré dans le sang du fait d'une infection par la syphilis.

3. Réactif selon la revendication 1, dans lequel l'anticorps anti-phospholipide servant d'analyte est un anticorps anti-phospholipide généré dans le sang du fait du syndrome d'anticorps anti-phospholipide, qui est une maladie auto-immune.

4. Procédé pour doser un anticorps anti-phospholipide présent dans le sang en tant qu'analyte par réaction immunitaire avec un antigène phospholipidique qui est supporté sur un support insoluble, lequel procédé comprend l'incorporation d'un glycérophospholipide et d'une polyvinylpyrrolidone dans le système réactionnel immunitaire,
dans lequel le glycérophospholipide est dissous ou dispersé dans un tampon pour le système réactionnel et est une ou plusieurs espèce(s) choisie(s) dans l'ensemble constitué par l'acide phosphatidique, la phosphatidylcholine, le phosphatidylglycérol, la phosphatidyléthanolamine, et la phosphatidylsérine.

5. Procédé de dosage selon la revendication 4, dans lequel l'anticorps anti-phospholipide servant d'analyte est un anticorps anti-phospholipide de syphilis généré dans le sang du fait d'une infection par la syphilis.

6. procédé de dosage selon la revendication 4, dans lequel l'anticorps anti-phospholipide servant d'analyte est un anticorps anti-phospholipide généré dans le sang du fait du syndrome d'anticorps anti-phospholipide, qui est une maladie auto-immune.

Fig. 1

Fig. 2

Fig. 3

Fig. 4

Fig. 5

**Referential Example 3**

Fig. 6

**Referential Example 4**

Fig. 7

Referential Example 5

Fig. 8

Referential Example 6

Fig. 9

Fig. 10

Fig. 11

**Comparative Referential Example 1**

Fig. 12

[Example 1:PVP-K90,PG:0.43mg/mL]

Measurements($\Delta$Abs×10000)

| RU | 0 | 1 | 2 | 4 | 8 |
|------|-----|-----|------|------|------|
| 0day | 376 | 762 | 1206 | 2154 | 2953 |
| 3day | 351 | 700 | 1102 | 2110 | 2925 |
| 7day | 342 | 696 | 1096 | 2078 | 2890 |

Vs. day 0 %

| RU | 0 | 1 | 2 | 4 | 8 |
|------|-----|-----|-----|-----|-----|
| 0day | 100 | 100 | 100 | 100 | 100 |
| 3day | 93 | 92 | 91 | 98 | 99 |
| 7day | 91 | 91 | 91 | 96 | 98 |

Fig. 13

[Example 2:PVP-K90,PG:0.55mg/mL]

Measurements（ΔAbs×10000)

| RU | 0 | 1 | 2 | 4 | 8 |
|---|---|---|---|---|---|
| 0day | 375 | 757 | 1150 | 2144 | 2953 |
| 3day | 354 | 708 | 1094 | 2084 | 2906 |
| 7day | 380 | 713 | 1124 | 2080 | 2909 |

Vs. day 0 %

| RU | 0 | 1 | 2 | 4 | 8 |
|---|---|---|---|---|---|
| 0day | 100 | 100 | 100 | 100 | 100 |
| 3day | 94 | 93 | 95 | 97 | 98 |
| 7day | 101 | 94 | 98 | 97 | 98 |

Fig. 14

**[Example 3 : PVP-K90,PG : 0.64mg/mL]**

Measurements (△Abs × 10000)

| RU | 0 | 1 | 2 | 4 | 8 |
|------|-----|-----|------|------|------|
| 0day | 371 | 715 | 1131 | 2123 | 2902 |
| 3day | 389 | 717 | 1116 | 2089 | 2889 |
| 7day | 401 | 745 | 1128 | 2085 | 2870 |

Vs. day 0 %

| RU | 0 | 1 | 2 | 4 | 8 |
|------|-----|-----|-----|-----|-----|
| 0day | 100 | 100 | 100 | 100 | 100 |
| 3day | 105 | 100 | 99 | 98 | 100 |
| 7day | 108 | 104 | 100 | 98 | 99 |

Fig. 15

[Comparative Example 1: pullulan]

Measurements ($\triangle$Abs $\times$ 10000)

| RU | 0 | 1 | 2 | 4 | 8 |
|---|---|---|---|---|---|
| 0day | 326 | 430 | 681 | 1609 | 3084 |
| 3day | 230 | 309 | 496 | 1373 | 2846 |
| 7day | 234 | 300 | 450 | 1274 | 2875 |

Vs. day 0 %

| RU | 0 | 1 | 2 | 4 | 8 |
|---|---|---|---|---|---|
| 0day | 100 | 100 | 100 | 100 | 100 |
| 3day | 71 | 72 | 73 | 85 | 92 |
| 7day | 72 | 70 | 66 | 79 | 93 |

Fig. 16

[Comparative Example 2: Lipidure]

Measurements ($\triangle$ Abs $\times$ 10000)

| RU | 0 | 1 | 2 | 4 | 8 |
|---|---|---|---|---|---|
| 0day | 288 | 395 | 695 | 1736 | 3245 |
| 3day | 314 | 416 | 689 | 1705 | 3061 |
| 7day | 516 | 614 | 874 | 1816 | 3080 |

Vs. day 0 %

| RU | 0 | 1 | 2 | 4 | 8 |
|---|---|---|---|---|---|
| 0day | 100 | 100 | 100 | 100 | 100 |
| 3day | 109 | 105 | 99 | 98 | 94 |
| 7day | 179 | 156 | 126 | 105 | 95 |

Fig. 17

| [Comparative Example 3: PEG70000] | | | | | |
|---|---|---|---|---|---|
| Measurements （△Abs×10000) | | | | | |
| RU | 0 | 1 | 2 | 4 | 8 |
| 0day | 329 | 361 | 522 | 1256 | 2715 |
| 3day | 271 | 293 | 402 | 1007 | 2315 |
| 7day | 287 | 284 | 411 | 998 | 2232 |
| Vs. day 0 % | | | | | |
| RU | 0 | 1 | 2 | 4 | 8 |
| 0day | 100 | 100 | 100 | 100 | 100 |
| 3day | 82 | 81 | 77 | 80 | 85 |
| 7day | 87 | 79 | 79 | 79 | 82 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H07103980 A
- JP H06148193 A

- JP H10282096 A

**Non-patent literature cited in the description**

- *Public Health Reports,* 1960, vol. 75, 985-988